# EUROPEAN PATENT SPECIFICATION

(11) **EP 4 518 836 B1**
(45) Date of publication and mention of the grant of the patent: **07.10.2026**
(21) Application number: 23725618.5
(22) Date of filing: 05.05.2023
(51) Int. Cl.: A61K 9/00, A61K 47/38

(54) **INSERT FOR THE TREATMENT OF DRY EYES**
EINSATZ ZUR BEHANDLUNG VON TROCKENEM AUGE
INSERT POUR LE TRAITEMENT DES YEUX SECS

(30) Priority: 06.05.2022 BE 202205346; 06.05.2022 BE 202205347
(43) Date of publication of application: 12.03.2025
(73) Proprietor: Eyed Pharma, 4102 Seraing (BE)
(72) Inventor: WIJZEN, Fabienne, 4140 Sprimont (BE); HOFFELINCK, Ingeborg, 9340 Smetlede (BE); ALAIMO, David, 4102 Seraing (BE)
(74) Representative: Brantsandpatents bv
(86) International application number: PCT/EP2023/061965
(87) International publication number: WO 2023/214027

(56) References cited:
- CN-A- 113 795 242
- US-A- 4 343 787
- US-A- 4 730 013
- US-A1- 2008 193 407
- US-A1- 2011 129 516

## Description

### FI ELD OF THE INVENTI ON

The present invention relates to ophthalmic inserts and more particularly to those for the treatment of dry eye.

### BACKGROUND

Dry eye, also known as dry eye syndrome or keratoconjunctivitis sicca, although seemingly trivial, is in practice a poorly solved problem that greatly affects the quality of life of affected patients. If left untreated, the disease can lead to blindness. The disease is multifactorial and is reflected by poor tear film homeostasis, instability, hypertonicity, inflammation and damage to the ocular surface, and aberrant nerve sensitivity.

Two causes are recognised, insufficient tear production and excessive evaporation, the latter being much more common.

Current treatments include the application of artificial tears, corticosteroids or immunodepressants (e.g. Cyclosporine A) in solution, administered as drops.

However, these treatments are heavy over time. In particular, when the disease is at a more advanced stage, the application of drops several times a day is necessary, for example 6 times, which is particularly delicate for very old people, whereas the disease at its advanced stage is often found in older people.

Instead of regularly applied drops, inserts based on hydrophilic bioresorbable polymers have been developed in the past and even marketed.

Patent US3845201 describes an alginate-based insert in synergy with pilocarpine.

Patent US4343787 describes a hydroxypropyl cellulose (HPC) based insert for the treatment of dry eye. This patent covers an insert that was marketed under the name Lacrisert^{®}.

Patent GB1485149 also describes an HPC-based insert reinforced with an active agent.

Patent US4730013 describes an insert based on hydroxypropyl methyl cellulose (HPMC), or HPC, which may include compounds such as polyvinyl alcohol, mannitol or glycerine. The aim is to reduce the phenomenon of blurred vision, which is all too common when applying Lacrisert^{®}.

Patent US6217896 describes the advantage associated with a particular geometry of the insert.

These various publications show, in fact, that the best product that has been developed is Lacrisert^{®}, and that various approaches have been tried to improve it, without major success.

### SUMMARY OF THE INVENTI ON

The present invention and embodiments thereof serve to provide a solution to one or more of the above-mentioned disadvantages. To this end, the present invention relates to an ophthalmic according to claim 1. In a second aspect, the present invention relates to an ophthalmic insert for use in preventing or treatment of dry eye syndrome. In a third aspect the present invention relates to a method according to claim 9.

The inventors have noticed that the problems of simple ophthalmic inserts made of hydroxypropyl cellulose (HPC) can be overcome by adding at least one functional molecule. This can be compounds with an osmoprotective action and/or compounds to modify the viscosity (hereafter described as "viscosity agent"). This can also be plasticizers or humectants.

The application provides an improved ophthalmic insert which is effective in the treatment of dry eye but overcomes the above-mentioned problems. A relatively high molecular weight HPC can still be employed while reducing the issues related to blurred vision. This insert allows to control the viscosity in the eye, but also the resorption kinetics of the insert

Furthermore, the application provides an improved manufacturing method for ophthalmic inserts which allows incorporation of functional molecules aiding in overcoming the above-mentioned problems.

### DETAI LED DESCRIPTION OF THE INVENTION

The present invention concerns a bioresorbable ophthalmic insert.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
In the context of the present invention, "ophthalmic insert" is preferably understood to mean a sterile solid or semi-solid structure of appropriate size and shape, intended to be inserted into the conjunctival cul-de-sac for action on the eye. Advantageously, such inserts have a mass of a few milligrams, for example between 1 and 50 milligrams, preferably between 2 and 40 milligrams, or between 3 and 30 milligrams, or between 4 and 20 milligrams or between 5 and 10 milligrams and, preferably, an elongated structure (cylinder, stick), which may be curved, domed or rounded at the ends.

Preferably the insert has an elongated shape such as a stick, a cylinder, an elliptical cylinder or a deformed crescent-shaped cylinder, the insert may also be shaped like a "grain of rice" (i.e. a stick with (substantially) hemispherical, rather than flat, ends) or a "rugby ball" (i.e. oval, ellipsoid).

The maximum dimension (length) is preferably less than 7 mm, preferably less than 5 mm, most preferably less than 3 mm. In the case of a cylinder, the maximum dimension is preferably understood to be the length. In the case of an ellipsoid, the maximum dimension is preferably understood to be the major axis. The secondary dimension is preferably understood as the diameter of the cylinder or the major axis of the elliptical cylinder or the minor axis of the ellipsoid. The secondary dimension is preferably less than 2 mm, preferably less than 1.5 mm, more preferably less than 1 mm. In the case of a cylinder with hemispherical "rice grain" ends, the primary dimension is preferably the length of the cylinder plus the sum of the two radii of the two end hemispheres.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g.,* any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

This invention relates to a non-toxic solid water-soluble polymer ophthalmic insert useful in the treatment of dry eye syndrome. Thus, a first aspect of the invention relates to a bioresorbable ophthalmic insert as disclosed in claim 1.

The insert may be placed into the cul-de-sac of the eye to obtain long term release of "artificial tears". The polymer used to form the inserts of this invention is preferably hydroxypropyl cellulose.

Hydroxypropyl cellulose is available in in several different viscosity grades, which is dependent on the molecular weight of the polymer chains, all of which are suitable in the preparation of these inserts. Thus, the product sold by Ashland, Inc. of Wilmington, Del. under the name KLUCEL which are intended for food or pharmaceutical use are particularly useful in preparing the inserts of this invention.

The ophthalmic insert comprises hydroxypropyl cellulose (HPC). Said HPC preferably has a molecular weight of at least at least 800,000 Da and of at most 1200,000 Da. HPC has a molecular weight of between 800,000 and 1200,000 Da.

The ophthalmic insert comprises further a functional molecule. The term "functional molecule" refers to any molecule which aids in achieving a biological or chemical effect. A functional molecule in this application is preferably a viscosity agent or an osmo-protectant.

The functional molecule is sodium hyaluronate. The functional molecule can further include molecules selected from the group consisting of L-carnitine, betaine, trehalose (α-α trehalose), erythritol and/or mixtures thereof.

In further embodiments, the functional molecule may also include ectoin. Ectoin has been shown to have excellent water-binding and moisturizing properties, which could help alleviate the symptoms of dry eye disease. When used in eye inserts, Ectoin can form a protective layer over the surface of the eye, helping to retain moisture and reduce dryness and irritation. It can also help to stabilize the lipid layer of the tear film, which can further reduce the evaporation of tears and increase lubrication. Moreover, Ectoin is a biocompatible and safe substance, with no known adverse effects, making it an attractive option for use in eye inserts for dry eye disease. It can be used alone or in combination with other compounds to improve the effectiveness of the eye inserts.

In other or further embodiments, the functional molecule may also include dextran. In the case of dry eye disease, Dextran can be used in eye inserts to help retain moisture on the surface of the eye and reduce dryness and irritation. The increased viscosity provided by Dextran helps the eye inserts to adhere better to the ocular surface and prolong their residence time. Moreover, Dextran can also act as a barrier to prevent the loss of moisture from the tear film and increase the stability of the tear film. This can help reduce the evaporation of tears and improve the overall lubrication of the eye. Dextran is a biocompatible and safe substance that has been extensively studied and used in medical applications for many years. It is also non-toxic and non-immunogenic, making it a safe option for use in eye inserts for dry eye disease.

In other or further embodiments, the functional molecule may also include one or more polyols. In the case of dry eye disease, polyols can be used in eye inserts to help retain moisture on the surface of the eye and reduce dryness and irritation. Like Dextran, polyols can act as a barrier to prevent the loss of moisture from the tear film, increase the stability of the tear film, and improve the overall lubrication of the eye. Polyols can also help to reduce inflammation in the eye. Dry eye disease can lead to inflammation of the ocular surface, which can exacerbate the symptoms of the condition. Polyols have been shown to have anti-inflammatory properties, which can help to reduce inflammation and improve the overall health of the ocular surface. Moreover, polyols have good biocompatibility, low toxicity, and high water solubility, which makes them a safe and effective component for use in eye inserts for dry eye disease. Examples of polyols suitable for use in the eye insert are glycerol, polyethylene glycol, polysorbate, polyvinyl alcohol, povidone, propylene glycol, or combinations thereof.

In other or further embodiments, the functional molecule may also include carbomer. In the case of dry eye disease, Carbomer can be used in eye inserts to improve the retention time and lubrication of the inserts on the ocular surface. Carbomer can increase the viscosity of the inserts, which helps them to adhere to the ocular surface and prolong their residence time. This can improve the effectiveness of the inserts by allowing them to release their active ingredients over a longer period of time. Carbomer can also help to stabilize the tear film and reduce the evaporation of tears, which can help to alleviate the symptoms of dry eye disease. Carbomer forms a gel-like layer on the surface of the eye, which can help to prevent the loss of moisture from the tear film and improve the overall lubrication of the eye.

In other or further embodiments, the functional molecule may also include an antioxidant.

In the case of dry eye disease, oxidative stress and inflammation are known to contribute to the development and progression of the condition. Antioxidants such as Vitamin C, Vitamin E, and N-acetylcysteine have been shown to have anti-inflammatory and antioxidant properties, which can help to reduce inflammation and protect the ocular surface from damage. When used in eye inserts, antioxidants can help to improve the health and function of the ocular surface, reduce the symptoms of dry eye disease, and prevent further damage. Moreover, antioxidants can help to enhance the stability of other active ingredients in the eye inserts, improving their overall effectiveness. Antioxidants can also improve the biocompatibility and safety of the inserts by reducing the risk of adverse reactions. Examples of antioxidant suitable for use in the eye insert are vitamin C, vitamin E, and N-acetylcysteine.

Carbomer and polyols may work together synergistically to increase the viscosity and retention time of the eye inserts, as well as improve the lubrication and moisture retention on the ocular surface. Combining these two components may result in a more effective and longer-lasting eye insert.

Additionally, incorporating antioxidants such as vitamin E and vitamin C in eye inserts along with ectoin or dextran may have a synergistic effect. Ectoin and dextran have been shown to reduce inflammation and stabilize the tear film, while antioxidants can protect the ocular surface from damage caused by free radicals. Combining these molecules may provide a more comprehensive and effective treatment for dry eye disease by addressing both the underlying causes of the condition as well as the symptoms.

Furthermore, carbomer can be combined with ectoin or dextran to improve their adhesion to the ocular surface and prolong their residence time, allowing for a sustained release of the active ingredients. The addition of antioxidants to this combination may provide further protection against oxidative stress and inflammation, leading to a more effective and longer-lasting treatment.

When combined with the molecules discussed above, trehalose or sodium hyaluronate can provide synergistic benefits in the treatment of dry eye disease. For example, combining trehalose and ectoin may have a synergistic effect in reducing inflammation and stabilizing the tear film. The presence of sodium hyaluronate with this combination improves the lubrication and hydration of the ocular surface, leading to a more comprehensive and effective treatment.

Moreover, incorporating antioxidants such as vitamin C and vitamin E with trehalose or sodium hyaluronate may provide additional protection against oxidative stress and inflammation, leading to improved outcomes in the treatment of dry eye disease.

Said sodium hyaluronate is preferably cross-linked sodium hyaluronate. Cross-linked sodium hyaluronate is a more viscoelastic material, with a non-Newtonian behavior. As a consequence, the increased viscoelasticity of cross-linked sodium hyaluronate determines a greater stability, a better resistance to degradation in stress conditions, and also a greater resistance to the enzymatic degradation by hyaluronidase. It is well tolerated in vitro and in vivo and exhibits longer resistance on the ocular surface and a reduction of dry eye symptoms on patients affected by dry eye disease.

Cross-linking techniques generally involve the primary and secondary hydroxyl groups, the carboxyl group, and the N-acetyl group. The hydroxyl group may be cross-linked via an ether linkage, and the carboxyl group via an ester linkage. Sodium hyaluronate may also be treated with acid or base to obtain free amino groups, a process referred to as deacetalization. These amino groups may be cross-linked via an amide, imino-, or secondary amino bond. Cross-linking reactions can be achieved under neutral, acidic, and alkaline conditions. Auto-cross-linking is based on the property of sodium hyaluronate to aggregate with itself, which is partly associated with bonding between its hydrophobic patches. Other cross-linking techniques include cross-linking with polyfunctional epoxides or with glutaraldehyde and with carbodiimides. Among these cross-linking agents, 1-ethyl-3-(3-dimethylaminopropyl)carbodiimide hydrochloride (EDC) is preferable because it can induce crosslinking of biomaterials without taking part in. EDC changes to water-soluble urea derivatives that have very low cytotoxicity. EDC represents in this sense a potential biopolymer cross-linker for the fabrication of various chemically modified carriers/scaffolds for ocular tissue engineering.

Said cross-linked sodium hyaluronate preferably has a degree of crosslinking between 0.01 and 10%, more preferably between 0.01 and 5%, even more preferably between 0.1 and 5%. The term "degree of cross-linking" refers to the moles of HA disaccharides actually involved in cross-linking in relation to the total moles of HA disaccharides.

The ophthalmic insert comprises:
- hydroxypropyl cellulose (HPC), said HPC preferably has a molecular weight of between 800,000 and 1,200,000 Da; and
- sodium hyaluronate, wherein said sodium hyaluronate has a particle size characterized by a D90 of at most 100 µm.

The inventors have found that sodium hyaluronate also acts as a humectant (hygroscopic agents that facilitate the retention of water) in the insert and can bind water multiple times the amount of its weight. It provides additional hydration to the cornea. Sodium hyaluronate may also promote corneal epithelial would healing by promoting the migration of corneal epithelial cells.

Said functional molecule is preferably grounded. The inventors found that grounding the functional molecule, preferably the sodium hyaluronate, resulted in a regular dispersion of the functional molecule, preferably the sodium hyaluronate, charge in the HPC matrix. The term "grounding" refers to the reducing of the particle size. The inventors unexpectedly found that grounding the functional molecule, preferably the sodium hyaluronate, resulted in a better control of the insert diameter and a more stable extrusion process (less irregularities in the stretching).

The functional molecule preferably has a particle size characterized by a D90 of at most most 100 µm.

The inventors found that this particle size ensures the homogeneity of the formulation and avoid irregularity during the stretching of the obtained filament (extrudate) due to the presence of bigger particles. Furthermore, this particle size showed ideal release conditions.

The inventors found that this particle size resulted in a better control of the filament (extrudate) diameter and a more stable extrusion process. Bigger particles create irregularities in the filament stretching during extrusion and instabilities in the extrusion process.

The functional molecule preferably has a particle size characterized by a D90 of at least 1 µm, more preferably at least 5 µm, even more preferably at least 10 µm, even more preferably at least 15 µm, even more preferably at least 20 µm.

The granulometry of the functional molecule is preferably not too small in order to avoid an acceleration of the functional molecule dissolution and consequently a decrease of the in use time in the eye.

In the context of the present invention, the degree of substitution of the HPC is preferably higher than 1, preferably higher than 2, preferably 2.5 (approx.) or even higher, e.g. approx. 3 or even approx. 4: number of hydroxypropyl molecules per glucose residue. A number greater than 2.5 means that some hydroxypropyl residues have been grafted onto other hydroxypropyl residues, and not directly onto the glucose chain forming the cellulose.

The HPC (first cellulose derivative) has a molecular weight between 800,000 and 1,200,000 Da. This, compared to HPCs with a lower molecular weight and/or compared to HPCs with a higher molecular weight, allows for a somewhat prolonged effect, and prevents the "blurred vision" phenomenon from being too pronounced.

Preferably, the functional molecule is incorporated in a mass content of at least 5% by weight of the insert, preferably at least 10, 15, 20, 25, 30, 35 or even about 40% by weight of the insert.

Preferably the sodium hyaluronate is incorporated in a mass content of at least 5% by weight of the insert, preferably at least 10, 15, 20, 25 or even about 30% by weight of the insert.

Preferably, the functional molecule, preferably sodium hyaluronate, is present in the insert in an amount between 1 and 99% by weight of the insert, preferably in an amount between 1 and 75% by weight of the insert, more preferably in an amount between 1 and 50% by weight of the insert, even more preferably in an amount between 1 and 25% by weight of the insert, even more preferably in an amount between 1 and 10% by weight of the insert, even more preferably in an amount between 2 and 8% by weight of the insert, even more preferably in an amount between 3 and 7% by weight of the insert, and even more preferably in an amount between 4 and 6% by weight of the insert.

In the context of the present invention, in addition to sodium hyaluronate, salts of hyaluronate with other cations are possible, provided that the salt remains sufficiently soluble in the tear fluid (water) and is not toxic. Also, some residues can be protonated, so that the term "sodium hyaluronate" is used in this invention to cover hyaluronic acid. When mass contents are associated with hyaluronate or hyaluronic acid, these are preferably expressed as sodium hyaluronate equivalents.

The ophthalmic inserts of this invention can also comprise plasticizers, buffering agents and preservatives. The invention is therefore also directed to ophthalmic inserts comprising these materials along with HPC. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol propane, di and tripropylene glycol, hydroxypropyl sucrose and the like. The inventors have found that the plasticizer can also be trehalose.

A preferred plasticizer which may be present in the ophthalmic insert is trehalose. Trehalose is at its melted state at the use extrusion temperature and has been found to help decreasing the processing temperature of the HPC (polysaccharide) and consequently decrease the risk of degradation of functional molecule, preferably the sodium hyaluronate, during the extrusion process.

A preferred plasticizer which may be present in the ophthalmic insert is polyethylene glycol (PEG). Different grades of PEG are suitable such as PEG 1450, PEG 3350 and PEG 8000.

The inventors found that addition of a plasticizer, preferably PEG, reduces the rigidity of the product during processing at low extrusion temperatures (to try to avoid HA degradation). The plasticizer results in the obtained extruded filaments (rod) to show a less excessively variable diameter.

In a preferred embodiment, the ophthalmic insert comprises a plasticizer, preferably PEG, in an amount between 0.5 and 10 % by weight of the insert, preferably in an amount between 0.5 and 5 % by weight of the insert, more preferably in an amount between 0.5 and 4.5% by weight of the insert, even more preferably in an amount between 0.5 and 4% by weight of the insert, and even more preferably in an amount between 0.5 and 2.5% by weight of the insert.

In a preferred embodiment, the ophthalmic insert comprises a plasticizer, preferably trehalose, in an amount between 1 and 40% by weight of the insert, preferably in an amount between 5 and 35% by weight of the insert, more preferably in an amount between 10 and 30% by weight of the insert, even more preferably in an amount between 15 and 25% by weight of the insert.

In a particularly preferred embodiment, the ophthalmic insert comprises:
- hydroxypropyl cellulose (HPC), said HPC preferably has a molecular weight of between 800,000 and 1,200,000 Da; and
- sodium hyaluronate, wherein said sodium hyaluronate has a particle size characterized by a D90 of at most 100 µm;
- a plasticizer, preferably polyethylene glycol.

In a further particularly preferred embodiment, the ophthalmic insert consists of:
- hydroxypropyl cellulose (HPC), said HPC has a molecular weight of between 800,000 and 1,200,000 Da; and
- sodium hyaluronate, wherein said sodium hyaluronate has a particle size characterized by a D90 of at most 100 µm;
- a plasticizer, preferably a polyethylene glycol;
- impurities and/or additives in an amount up to 5 percent by weight of the insert, preferably in an amount up to 3 percent by weight of the insert, more preferably in an amount up to 2 percent by weight of the insert, more preferably in an amount up to 1 percent by weight of the insert, more preferably in an amount up to 0.5 percent by weight of the insert, more preferably in an amount up to 0.1 percent by weight of the insert.

In an even further particularly preferred embodiment, the ophthalmic insert consists of:
- sodium hyaluronate, wherein said sodium hyaluronate has a particle size characterized by a D90 of at most 100 µm, in an amount of between 2 and 8% by weight of the insert;
- a plasticizer, preferably a polyethylene glycol, in an amount of between 0.5 and 5% by weight of the insert;
- impurities and/or additives in an amount up to 5% by weight of the insert, preferably in an amount up to 3% by weight of the insert, more preferably in an amount up to 2% by weight of the insert, more preferably in an amount up to 1% by weight of the insert, more preferably in an amount up to 0.5% by weight of the insert, more preferably in an amount up to 0.1% by weight of the insert, more preferably no impurities and/or additives; and
- remainder hydroxypropyl cellulose (HPC), said HPC has a molecular weight of between 800,000 and 1,200,000 Da.

"Remainder HPC" means the insert comprises HPC, in an amount to achieve a total insert composition of 100 by weight of the insert.

In another particularly preferred embodiment, the ophthalmic insert comprises:
- hydroxypropyl cellulose (HPC), said HPC has a molecular weight of between 800,000 and 1,200,000 Da;
- sodium hyaluronate, wherein said sodium hyaluronate has a particle size characterized by a D90 of at most 100 µm; and
- a plasticizer, preferably trehalose (α-α trehalose).

In a further particularly preferred embodiment, the ophthalmic insert consists of:
- hydroxypropyl cellulose (HPC), said HPC has a molecular weight of between 800,000 and 1,200,000 Da;
- sodium hyaluronate, wherein said sodium hyaluronate has a particle size characterized by a D90 of at most 100 µm;
- a plasticizer, preferably trehalose (α-α trehalose); and
- impurities and/or additives in an amount up to 5 percent by weight of the insert, preferably in an amount up to 3 percent by weight of the insert, more preferably in an amount up to 2 percent by weight of the insert, more preferably in an amount up to 1 percent by weight of the insert, more preferably in an amount up to 0.5 percent by weight of the insert, more preferably in an amount up to 0.1 percent by weight of the insert, more preferably no impurities and/or additives.

In an even further particularly preferred embodiment, the ophthalmic insert consists of:
- sodium hyaluronate, wherein said sodium hyaluronate has a particle size characterized by a D90 of at most 100 µm, in an amount of between 2 and 8% by weight of the insert;
- a plasticizer, preferably a trehalose, in an amount of between 10 and 30% by weight of the insert;
- impurities and/or additives in an amount up to 5% by weight of the insert, preferably in an amount up to 3% by weight of the insert, more preferably in an amount up to 2% by weight of the insert, more preferably in an amount up to 1% by weight of the insert, more preferably in an amount up to 0.5% by weight of the insert, more preferably in an amount up to 0.1% by weight of the insert, more preferably no impurities and/or additives; and
- remainder hydroxypropyl cellulose (HPC), said HPC has a molecular weight of between 800,000 and 1,200,000 Da.

In another particularly preferred embodiment, the ophthalmic insert comprises:
- hydroxypropyl cellulose (HPC), said HPC has a molecular weight of between 800,000 and 1,200,000 Da; and
- a combination of sodium hyaluronate and trehalose (α-α trehalose), wherein said sodium hyaluronate has a particle size characterized by a D90 of at most 100 µm.

In a further particularly preferred embodiment, the ophthalmic insert consists of:
- hydroxypropyl cellulose (HPC), said HPC has a molecular weight of between 800,000 and 1,200,000 Da;
- a combination of sodium hyaluronate and trehalose (α-α trehalose), wherein said sodium hyaluronate has a particle size characterized by a D90 of at most 100 µm; and
- impurities and/or additives in an amount up to 5 percent by weight of the insert, preferably in an amount up to 3 percent by weight of the insert, more preferably in an amount up to 2 percent by weight of the insert, more preferably in an amount up to 1 percent by weight of the insert, more preferably in an amount up to 0.5 percent by weight of the insert, more preferably in an amount up to 0.1 percent by weight of the insert, more preferably no impurities and/or additives.

In an even further particularly preferred embodiment, the ophthalmic insert consists of:
- hydroxypropyl cellulose (HPC), said HPC has a molecular weight of between 800,000 and 1,200,000 Da, in an amount of at least 25% by weight of the insert; and
- a combination of sodium hyaluronate and trehalose, wherein said sodium hyaluronate has a particle size characterized by a D90 of at most 100 µm, in an amount of between 5 and 75% by weight of the insert;
- impurities and/or additives in an amount up to 5% by weight of the insert, preferably in an amount up to 3% by weight of the insert, more preferably in an amount up to 2% by weight of the insert, more preferably in an amount up to 1% by weight of the insert, more preferably in an amount up to 0.5% by weight of the insert, more preferably in an amount up to 0.1% by weight of the insert, more preferably no impurities and/or additives.

In another particularly preferred embodiment, the ophthalmic insert comprises:
- sodium hyaluronate, wherein said sodium hyaluronate has a particle size characterized by a D90 of at most 100 µm, in an amount of between 2 and 8% by weight of the insert,
- trehalose, in an amount of between 10 and 30% by weight of the insert,
- impurities and/or additives in an amount up to 5% by weight of the insert, preferably in an amount up to 3% by weight of the insert, more preferably in an amount up to 2% by weight of the insert, more preferably in an amount up to 1% by weight of the insert, more preferably in an amount up to 0.5% by weight of the insert, more preferably in an amount up to 0.1% by weight of the insert, more preferably no impurities and/or additives; and
- remainder hydroxypropyl cellulose (HPC), said HPC has a molecular weight of between 800,000 and 1,200,000 Da.

### Trehalose

In a preferred embodiment, the ophthalmic insert comprises sodium hyaluronate and trehalose (α-α trehalose).

The inventors have found that the use of trehalose in the formulation has a double effect. On one side, trehalose is known for its efficacy on the treatment of dry eye, but the inventors have unexpectedly found that the use of trehalose in the extrusion blend could help decrease the extrusion temperature as it could act as a plasticizer.

In this embodiment, preferably both functional molecules have a particle size characterized by a D90 of at most 100 µm.

The inventors found that this particle size ensures the homogeneity of the formulation and avoid irregularity during the stretching of the obtained filament (extrudate) due to the presence of bigger particles. Furthermore, this particle size showed ideal release conditions.

The inventors found that this particle size resulted in a better control of the filament diameter and a more stable extrusion process. Bigger particles create irregularities in the filament stretching during extrusion and instabilities in the extrusion process.

The functional molecule preferably has a particle size characterized by a D90 of at least 1 µm, more preferably at least 5 µm, even more preferably at least 10 µm, even more preferably at least 15 µm, even more preferably at least 20 µm.

The granulometry of the functional molecule is preferably not too small in order to avoid an acceleration of the functional molecule dissolution and consequently a decrease of the in use time in the eye.

In the context of the present invention, the trehalose is preferably α-α trehalose in any degree of hydration, preferably α-α trehalose dihydrate. However, in the context of the present invention, the mass percentages are advantageously expressed by relating the trehalose equivalents to α-α trehalose dihydrate (although, in practice, α-α trehalose in other degrees of hydration (or anhydrous) is potentially present in the insert).

However, unlike drops, the inventors found that the addition of trehalose, ideally more than 1% trehalose by weight of the insert, allowed for increased and/or prolonged efficacy for the patient. In turn, this allowed for less frequent application of the insert, or the application of smaller inserts, thereby reducing the known deleterious effects of "blurry vision".

Thus, preferably, the ophthalmic insert comprises the functional molecule, in an amount of between 2 and 40%, preferably between 3 and 30%, more preferably between 4 and 20%, most preferably between 5 and 15% by weight of the insert.

Preferably, sodium hyaluronate and trehalose, are present in the insert in a total amount between 1 and 75% by weight of the insert, preferably in an amount between 1 and 50% by weight of the insert, more preferably in an amount between 5 and 50% by weight of the insert, even more preferably in an amount between 10 and 40% by weight of the insert.

As described above, the inventors have noticed that the functional molecule, or a mixture of functional molecules described above, has an osmo-protective effect, which is advantageous.

Advantageously, the ophthalmic insert comprises both sodium hyaluronate and trehalose, preferably sodium hyaluronate, in an amount of at least 1% (preferably at least 2, 3, 4, 5, 10, 15%) by weight of the insert, and trehalose, in an amount of at least 1% ( preferably at least 2, 3, 4, 5, 10, 15% ) by weight of the insert.

Preferably, the trehalose (α-α trehalose) is incorporated in a mass content of at least 5% by weight of the insert, preferably at least 10, 15, 20, 25, 30, 35 or even about 40% by weight of the insert.

Preferably, the sodium hyaluronate and the trehalose (α-α trehalose) are incorporated in a total mass content of at least 5% by weight of the insert, preferably at least 10, 15, 20, 25, 30, 35 or even about 40% by weight of the insert.

In an embodiment, the bioresorbable ophthalmic insert comprises at least 25% by weight of hydroxypropyl cellulose (HPC), said HPC having a molecular weight greater than 800,000 Da, and between 1 and 50% by weight sodium hyaluronate and a functional molecule selected from the group consisting of L-carnitine, betaine, trehalose (α-α trehalose), erythritol and/or mixtures thereof.

### Second cellulose derivative

The functional molecule may also be a viscosity agent. In the context of the present invention, preferably the term "viscosity agent" refers primarily to sodium hyaluronate and/or the second cellulose derivative (see below).

In the context of the present invention, advantageously, the second cellulose derivative and the hyaluronic acid/hyaluronate are viscosity agents, preferably conferring a non-Newtonian fluid property to the tear film. This allows to control the viscosity in the eye, but also the resorption kinetics of the insert. Indeed, the inventors have noted that the use of the first cellulose derivative, HPC of the size described above, represents a compromise capable of minimising the "blurred vision" phenomenon. However, a more marked reduction is not possible, even by optimising the physico-chemical characteristics of the first cellulose derivative, as opposed to incorporating a second cellulose derivative and/or sodium hyaluronate.

Preferably, the second cellulose derivative is present at a mass content of between 10 and 60% by weight of the insert, preferably between 15% and 50% by weight of the insert, more preferably between 20 and 40% by weight of the insert, for example between 25 and 30% by weight of the insert. Indeed, contents that are too high dilute the HPC content, or even the functional molecule if it is present (see below), while contents that are too low will not allow sufficient improvement.

Preferably, the ophthalmic insert comprises a second cellulose derivative different from the HPC (first cellulose derivative) having a molecular weight of between 800,000 and 1200,000 Da, this second cellulose derivative being selected from HPC of different molecular weight, hydroxypropyl methyl cellulose (HPMC), carboxy methyl cellulose (CMC), methyl cellulose and mixtures thereof. This second cellulose derivative is advantageously present when the HPC having a molecular weight between 800,000 and 1200,000 Da is incorporated in the insert only in proportions between 25 and 50% by weight of the insert. Preferably the HPC with a molecular weight of 800,000 to 1200,000 Da and the second cellulose derivative together make up at least 50% by weight of the insert, for example about 60% by weight of the insert.

Thus, preferably, the insert comprises at least 25% by weight of the insert of the first cellulose derivative and at least 25% by weight of the insert of the second cellulose derivative, or at least 30% of the first cellulose derivative and at least 20% by weight of the insert of the second cellulose derivative, or at least 35% by weight of the insert of the first cellulose derivative and at least 15% by weight of the insert of the second cellulose derivative, or at least 40% by weight of the insert of the first cellulose derivative and at least 10% by weight of the insert of the second cellulose derivative.

This allows the viscosity at the eye level to be controlled, but also the resorption kinetics of the insert. Indeed, the inventors have noticed that the use of HPC (first cellulose derivative) only meeting the size described above represents a compromise capable of reducing the phenomenon of "blurred vision" to a maximum. However, the incorporation of other molecules such as second cellulose derivatives, functional molecules (osmo-protecting agents and/or viscosity agents) advantageously allows a more marked reduction of this disturbing phenomenon.

Preferred HPMCs have a degree of methoxy substitution (DE) of between 20 and 30% (number of -OH residues of the glucose monomers substituted by a methyl group : total number of -OH residues of the glucose monomers), e.g. between 22 and 29% and/or a degree of hydroxypropyl substitution (DH) between 6 and 12%, preferably between 7 and 10%, e.g. (about) 8%, such as between 7.5 and 8.5% (number of -OH residues of the glucose monomers substituted with a hydroxypropyl group: total number of -OH residues of the glucose monomers). Depending on the molecular weight and degree of substitution, a high viscosity can be achieved at the patient's eye, while delaying resorption of the insert.

A HPMC with a viscosity of 4000 mPa.s (2%, by weight HPMC in an aqueous solution, 25°C) or higher is advantageous. Also HPMCs with a DE of about 29% and a DH of about 10% are preferred. Alternatively, HPMCs (of higher molecular weight) having an ED of 22% and a DH of 8% are advantageous.

Preferably, the second cellulose derivative is present at a content by weight of between 10 and 35% by weight of the insert, preferably between 15% and 30% by weight of the insert, more preferably between 20 and 25% by weight of the insert. Indeed, contents that are too high dilute the content of the first cellulose derivative (HPC), or even of the functional molecule, while contents that are too low will not allow sufficient improvement.

Preferably, when the second cellulose derivative is HPC, it has a molecular weight of between 100,000 and 750,000 Da, preferably between 200,000 and 700,000 Da, more preferably between 300,000 and 500,000 Da.

Alternatively, preferably, when the second cellulose derivative is HPC, it has a molecular weight between 1300,000 and 2500,000 Da.

In an embodiment, the bioresorbable ophthalmic insert comprises at least 25% by weight of the insert of a first cellulose derivative, being hydroxypropyl cellulose (HPC), said HPC having a molecular weight of between 800,000 and 1,200,000 Da, and between 10 and 75% by weight of the insert of a second cellulose derivative different from the first cellulose derivative, said second cellulose derivative being selected from HPC of different molecular weight, hydroxypropyl methyl cellulose (HPMC), carboxy methyl cellulose (CMC), methyl cellulose and mixtures thereof. Alternatively, the second cellulose derivative may advantageously be partially or fully substituted by hyaluronic acid and/or a hyaluronate, for example sodium hyaluronate.

Another aspect of the invention relates to a method for the manufacture of the above-mentioned insert.

The inventors have found that the incorporation of functional molecules such as sodium hyaluronate is not without difficulties. These functional molecules are not easy to incorporate into an HPC insert, due to the high temperatures encountered during the manufacturing process, but also because it is difficult to incorporate them into these small inserts in a homogeneous manner.

In a preferred embodiment, the method comprises the step of extruding a mixture comprising hydroxypropyl cellulose (HPC), said HPC having a molecular weight of between 800,000 and 1200,000 Da, and sodium hyaluronate, said sodium hyaluronate having a particle size characterized by a D90 of at most 100 µm, is performed, thereby obtaining an extrudate; and wherein said extrudate is cut to obtain ophthalmic inserts of desired dimensions.

The method comprises the step of extruding a mixture comprising hydroxypropyl cellulose (HPC), said HPC having a molecular weight of between 800,000 and 1,200,000 Da, and sodium hyaluronate, said sodium hyaluronate having a particle size characterized by a D90 of at most 100 µm and functional molecule selected from the group consisting of L-carnitine, betaine, trehalose ( α-α trehalose), erythritol and/or mixtures thereof, more preferably selected from the group consisting of trehalose (α-α trehalose), or a combination thereof.

The inventors have found that the addition of trehalose is advantageous as it is melted at the use extrusion temperature which allows a decreased processing temperature of the HPC (polysaccharide) and consequently decrease the risk of degradation of the functional molecule, preferably the sodium hyaluronate, during the extrusion process.

The functional molecule preferably has a particle size characterized by a D90 of at most 100 µm.

The inventors found that a minimized particle size results in a visually (with the eye) more homogeneous dispersion in the polymer matrix during the twin-screw extrusion.

The inventors found that this particle size ensures the homogeneity of the formulation and avoid irregularity during the stretching of the obtained filaments due to the presence of bigger particles. Furthermore, this particle size showed ideal release conditions.

The inventors found that this particle size resulted in a better control of the filament diameter and a more stable extrusion process. Bigger particles create irregularities in the filament stretching during extrusion and instabilities in the extrusion process. The functional molecule preferably has a particle size characterized by a D90 of at least 1 µm, more preferably at least 5 µm, even more preferably at least 10 µm, even more preferably at least 15 µm, even more preferably at least 20 µm.

The granulometry of the functional molecule is preferably not too small in order to avoid an acceleration of the functional molecule dissolution and consequently a decrease of the in use time in the eye.

In a preferred embodiment, said HPC and said functional molecule are dried prior to extrusion. The presence of water in the raw materials (HPC and functional molecule, and optionally second cellulose derivative) leads to the presence of bubbles during shaping and therefore to significant variability in the section of the product (filaments).

The inserts are advantageously cut in series, at the exit of the extrusion.

A preferred shape of the extruded insert is that of a cylinder or an elliptical cylinder. The dimensions are preferably as described above, i.e. preferably less than 7 mm, less than 5 mm or even less than 3 mm for the length and less than 2 mm, preferably less than 1.5 mm, preferably less than 1 mm for the diameter or major axis of the ellipse of the elliptical cylinder.

The extrusion is advantageously carried out via a twin-screw system. A second extrusion via a single screw system is advantageously carried out for a better control of the physical properties of the future insert (homogeneity, second dimension).The first and the second extrusion can alternatively be carried out by the same type of extruder. So, for example, the first and the second extrusion are carried out by a single-screw system or a twin-screw system.

Preferably, during extrusion, the temperature is kept as low as possible, i.e. at values below 250°C, preferably below 225°C, preferably below 200°C, preferably below 175°C, preferably below 150°C, or even below 125°C.

The low extrusion temperatures are advantageous to avoid degradation of the functional molecule, more specifically to avoid degradation of sodium hyaluronate.

In a preferred embodiment, the extrusion is carried out as a two-step extrusion. In this embodiment, a first extrusion is performed followed by a second extrusion.

In an even more preferred embodiment, said first extrusion step is carried out with a twin-screw extruder. In a further embodiment, said second extrusion step is carried out with a single-screw extruder.

The two-step extrusion process allows for the mixing and shaping to be performed in different steps of the process. Herein the first extrusion step can ensure the mixing of the raw materials, while the shaping of the product is carried out by the second extrusion step.

In a further preferred embodiment, the diameter of the die head used during the first extrusion step is greater than the diameter of the die head used during the second extrusion step. The use of a greater diameter in the first extrusion step allows for an easier extrusion with less browning due to blockage at the die head.

In a further embodiment the extrudate (filament) obtained after the first extrusion step can be pelletized. The term "pelletizing", as used herein, is the process of compressing or molding a material into the shape of a pellet, such as rounded, spherical, or cylindrical pellets. Pelletizing the extrudate allows easier feeding of the extrudate into the second extruder.

In a further preferred embodiment, the screw speed used during the first extrusion step is greater than the screw speed used during the second extrusion step.

Preferably the screw speed during the first extrusion step is between 70 and 110 rpm, more preferably between 80 and 100 rpm, more preferably between 85 and 95 rpm. Preferably the screw speed during the second extrusion step is between 1 and 40 rpm, more preferably between 5 and 30 rpm, more preferably between 7 and 25 rpm.

In a further preferred embodiment, the residence time of the product during the first extrusion step is smaller than the residence time during the second extrusion step.

The inventors have unexpectedly found that this allows for a first extrusion, wherein the raw materials are mixed without the risk of degradation of the functional molecule, followed by a second extrusion for shaping, wherein the functional molecule is protected by the HPC, and thus wherein the residence time can be raised.

Preferably the maximum extrusion temperature during the first extrusion step is at most 200°C, more preferably at most 190°C, more preferably at most 175°C. Preferably the maximum extrusion temperature during the second extrusion step is at least 100°C, more preferably at least 120°C, more preferably at least 140°C. Preferably the maximum extrusion temperature during the second extrusion step is at most 220°C, more preferably at most 210°C, more preferably at most 200°C.

In a further preferred embodiment, the maximum extrusion temperature during the first extrusion step is lower than the maximum extrusion temperature during the second extrusion step.

The inventors have unexpectedly found that this allows for a first extrusion, wherein the raw materials are mixed without the risk of degradation of the functional molecule, followed by a second extrusion for shaping, wherein the functional molecule is protected by the HPC, and thus wherein the extrusion temperature can be raised.

The inserts are advantageously cut in series, at the exit of the second extrusion.

In a particularly preferred embodiment, the method comprises the steps of:
- performing a first extrusion of of a mixture comprising hydroxypropyl cellulose (HPC), said HPC having a molecular weight of between 800,000 and 1200,000 Da, and sodium hyaluronate, said sodium hyaluronate having a particle size characterized by a D90 of at most 100 µm, and optionally a second cellulose derivative, thereby obtaining an extrudate;
- performing a second extrusion of said extrudate.

In a particularly preferred embodiment, the method comprises the steps of:
- performing a first extrusion of a mixture comprising hydroxypropyl cellulose (HPC), said HPC having a molecular weight of between 800,000 and 1200,000 Da, and sodium hyaluronate, said sodium hyaluronate having a particle size characterized by a D90 of at most 100 µm, and optionally a second cellulose derivative, or a combination thereof, thereby obtaining an extrudate;
- performing a second extrusion of said extrudate.

In a particularly preferred embodiment, the method comprises the steps of:
- performing a first extrusion of a mixture comprising hydroxypropyl cellulose (HPC), said HPC having a molecular weight of between 800,000 and 1,200,000 Da, and sodium hyaluronate, said sodium hyaluronate having a particle size characterized by a D90 of at most 100 µm, thereby obtaining an extrudate; performing a second extrusion of said extrudate.

In an embodiment, the method comprises the step of mixing at least 25% by weight of hydroxypropyl cellulose (HPC), said HPC having a molecular weight greater than 500,000 Da, and between 1 and 50% by weight of sodium hyaluronate, said sodium hyaluronate having a particle size characterized by a D90 of at most 100 µm, as well as any other components, and this mixture is extruded under specific temperature conditions compatible with the physico-chemical properties of the functional molecule(s), and the resulting structure is then cut to obtain the insert with the desired dimensions.

In another embodiment, the method comprises the step of mixing at least 25% by weight of hydroxypropyl cellulose (HPC), said HPC having a molecular weight of between 800,000 and 1,200,000 Da, and between 10 and 75% by weight of a viscosity agent being sodium hyaluronate, said sodium hyaluronate having a particle size characterized by a D90 of at most 100 µm and/or a second cellulose derivative different from the first cellulose derivative, this mixture is extruded under temperature conditions determined and compatible with the physico-chemical properties of the functional molecules and/or viscosity agents, then the structure obtained is cut to obtain the insert with the desired dimensions.

An alternative to extrusion is injection moulding (described but not part of the invention) which is advantageous for obtaining ellipsoidal or "rice grain" shapes, although the method is less automated. In this case, the components are homogeneously mixed (e.g. via twin-screw extrusion) at temperatures kept below 250°C, preferably below 225°C, preferably below 200°C, preferably below 175°C, preferably below 150°C, or even below 125°C, the mixture is pelletized, and then is injection molded.

Preferably the mould has a shape allowing an ellipsoid, a cylinder or an elliptical cylinder with hemispherical ends (grain of rice), preferably the major axis of the ellipsoid, the length of the cylinder or the length of the elliptical cylinder has a size of less than 7 mm, preferably less than 5 mm, preferably less than 3 mm and preferably the minor axis of the ellipsoid or the diameter of the cylinder or the major axis of the elliptical cylinder has a size of less than 2 mm, preferably less than 1.5 mm, preferably less than 1 mm.

The process (extrusion or injection) may further include the step of curving the insert.

An additional sterilisation step (of the extrusion or injection process) is advantageous. Of these, the use of ionising radiation, such as gamma irradiation, is preferred.

The invention is further described by the following non-limiting examples which further illustrate the invention, and are not intended to, nor should they be interpreted to, limit the scope of the invention.

### EXAMPLES

### Examples 1-4

HPC filaments comprising 10 w/w % sodium hyaluronate (HA) were produced. The HPC/HA mixtures were first homogenized using a Turbula-type device (3D mixer) at a speed of 66 rpm for 10 minutes. The mixtures were then processed using a twin-screw extruder. Table 1 below shows the initial parameters used for the extrusion of the HPC/HA mixture (twin screw extruder) after homogenization in the 3D mixer. The die head used for this test has an output diameter of 1.75 mm.

The characteristics of the extruder used are summarized below:
• Thermofisher Pharma 11mm extruder
• Changeable Design Screws
• Seven heating zones + one zone for the extrusion head
• Vent port
• Cooling belt
• (Pelletizer)

**TABLE 1**

| | | | Temperature Zone (°C) | | | | | |
|---|---|---|---|---|---|---|---|---|
| Ex. | HA (w/w%) | Screw speed | 1 | 2 | 3 | 4 | 5-6 | 7-8 |
| 1 | 10 | 66 rpm | 110 | 140 | 160 | 180 | 190 | 190 |
| 2 | 10 | 20 rpm | 110 | 140 | 160 | 180 | 190 | 200 |
| 3 | 10 | 70-90 rpm | 110 | 140 | 160 | 180 | 180 | 180 |
| 4 | 10 | 90 rpm | 110 | 140 | 160 | 175 | 175 | 175 |

During extrusion of the first mixture (example 1), the inventors observed the presence of large white hyaluronic acid grains irregularly dispersed on the surface of the filament.

In addition, sections of the collected filaments showed visible signs of sodium hyaluronate degradation when the filament is not pulled out of the die head fast enough. Extruder congestion was also observed, with an increase in torque and pressure at the die head, resulting in the device shutting down once the maximum operating pressure of the extruder was reached.

The parameters were adjusted (example 2) by decreasing the extrusion speed to 20 rpm and increasing the temperature of the last heating zones (T7 and T8) up to 200°C, which had the effect of decongesting the extruder but with a significant increase in the degradation of the rod produced (brown filament) linked to the higher residence time of the material in high temperature zones. Under the above conditions, bubble formation in the rod (blown filament) was also observed.

In example 3 and 4 the extrusion speed was increased, and the temperature reduced. The inventors found it was possible to produce filaments with no visible signs of degradation. The extrusion parameters of example 4 gave the best filaments.

### Example 5

HPC filaments comprising 10 w/w % HA were produced. The HPC/HA mixtures were first homogenized using a Turbula-type device (3D mixer) at a speed of 66 rpm for 10 minutes. The mixtures were then processed using a twin-screw extruder. Table 2 below shows the initial parameters used for the extrusion of the HPC/HA mixture (twin screw extruder) after homogenization in the 3D mixer. The die head used for this test has an output diameter of 3 mm.

**TABLE 2**

| | | | Temperature Zone (°C) | | | | |
|---|---|---|---|---|---|---|---|
| Ex. | HA (w/w%) | Screw speed | 1-3 | 4 | 5 | 6 | 7-8 |
| 5 | 10 | 90 rpm | 25 | 40 | 150 | 170 | 175 |

The resulting filament is white and easier to extrude than with the smaller die head (examples 1-4). The inventors found that advantageously regular and constant feeding of the product is ensured to avoid browning (degradation) of the filament, which occurs when the rod remains in contact with the die head for too long. The resulting rod, which has a larger cross-section than in examples 1-4, is also more rigid and therefore more difficult to stretch. The inventors found the homogeneity of the mixture between HA and HPC similar to example 4.

### Example 6-8

The filaments obtained in example 5 were converted into pellets and isolated. The pellets obtained were processed using a Scamex 12 mm single-screw extruder, in order to obtain regular filaments with a cross-section close to 1.27 mm.

The characteristics of the Scamex single-screw extruder used are summarized below:
• A feeder
• A simgle screw
▪ 12mm diameter
▪ Length: 21.4cm

• Die diameter : 2 mm
• Four heating zones.
• A Zumbach-type diameter measuring system (hundredth of a mm)
• (A cutting system)

**TABLE 3**

| | | | Temperature Zone (°C) | | | |
|---|---|---|---|---|---|---|
| Ex. | HA (w/w%) | Screw speed | 1 | 2 | 3 | 4 |
| 6 | 10 | 10 rpm | 140 | 160 | 175 | 180 |
| 7 | 10 | 15 rpm | 110 | 160 | 180 | 190 |
| 8 | 10 | 22 rpm | 110 | 165 | 190 | 200 |

The collected product is white and shows no visible signs of degradation, even when extruded at temperatures of around 200°C and at higher pressures (the single screw extruder operating under pressure unlike the twin screw extruder), contrary to the observations made when passing through the twin screw extruder. It would therefore appear that there is a protective effect once mixed with the HPC. The impact of shear during the mixing stage in the twin-screw extruder should also be taken into account in view of the observed product degradation, this effect being much more pronounced than during the subsequent extrusion stage in the single-screw extruder.

### Example 9

HPC filaments comprising 5 w/w % HA were produced. The HPC/HA mixtures were first homogenized using a Turbula-type device (3D mixer) at a speed of 66 rpm for 10 minutes. The mixtures were then processed using a twin-screw extruder. Table 4 below shows the initial parameters used for the extrusion of the HPC/HA mixture (twin screw extruder) after homogenization in the 3D mixer. The die head used for this test has an output diameter of 3 mm.

**TABLE 4**

| | | | Temperature Zone (°C) | | | | |
|---|---|---|---|---|---|---|---|
| Ex. | HA (w/w%) | Screw speed | 1-3 | 4 | 5 | 6 | 7-8 |
| 9 | 5 | 60 rpm | 25 | 40 | 150 | 170 | 175 |
| 10 | 5 | 60 rpm | 25 | 40 | 150 | 170 | 180 |
| 11 | 5 | 60 rpm | 25 | 40 | 150 | 170 | 185 |

When the temperatures of the T7 and T8 zones are fixed at 185°C (example 11), the filament turns brown on contact with the die head. At 180°C (example 10), brown areas are still observed when the filament is in contact with the die and the benefit is small on the quality of the draw compared to the 175°C temperature.

## Claims

1. A bioresorbable ophthalmic insert comprising:
- hydroxypropyl cellulose (HPC), said HPC having a molecular weight of between 800,000 and 1200,000 Da, and
- sodium hyaluronate, wherein said sodium hyaluronate has a particle size **characterized by** a D90 of at most 100 µm.

2. Ophthalmic insert according to claim 1, wherein the sodium hyaluronate is cross-linked sodium-hyaluronate.

3. Ophthalmic insert according to any of the previous claims, wherein the ophthalmic insert comprises said sodium hyaluronate, in an amount of between 2 and 8% by weight of the insert.

4. Ophthalmic insert according to any of the previous claims, comprising a second cellulose derivative, different from the first HPC having a molecular weight between 800,000 and 1200,000 Da, said second cellulose derivative being selected from HPC of different molecular weight, hydroxypropyl methyl cellulose (HPMC), carboxy methyl cellulose (CMC), methyl cellulose and mixtures thereof.

5. Ophthalmic insert according to any of the previous claims, wherein the ophthalmic insert comprises a functional molecule selected from the group consisting of L-carnitine, betaine, trehalose, erythritol and mixtures thereof.

6. Ophthalmic insert according to any of the previous claims, wherein the ophthalmic insert comprises trehalose.

7. Ophthalmic insert according to any of the preceding claims, wherein the ophthalmic insert comprises sodium hyaluronate, in an amount of between 2 and 8% by weight of the insert, and trehalose, in an amount of between 10 and 30% by weight of the insert.

8. An ophthalmic insert according to any of the claims 1-7 for use in prevention or treatment of dry eye syndrome.

9. A method for the manufacture of an ophthalmic insert, wherein an extrusion of a mixture comprising hydroxypropyl cellulose (HPC), said HPC having a molecular weight of between 800,000 and 1200,000 Da, and sodium hyaluronate, said sodium hyaluronate having a particle size **characterized by** a D90 of at most 100 µm, is performed, thereby obtaining an extrudate; and wherein said extrudate is cut to obtain ophthalmic inserts of desired dimensions.

10. Method according to claim 9, wherein the extrusion comprises the step of:
a. performing a first extrusion of a mixture comprising hydroxypropyl cellulose (HPC), said HPC having a molecular weight of between 800,000 and 1200,000 Da, and sodium hyaluronate, said sodium hyaluronate having a particle size **characterized by** a D90 of at most 100 µm, thereby obtaining an extrudate; and
b. performing a second extrusion of said extrudate.

11. Method according to claim 10, wherein the residence time of the product during the first extrusion step is smaller than the residence time during the second extrusion step.

12. Method according to claims 10-11, wherein the extrudate obtained after the first extrusion step is pelletized prior to the second extrusion step.

13. Method according to claims 9-11, wherein at least 25% by weight of hydroxypropyl cellulose (HPC), and between 2 and 8% by weight of sodium hyaluronate, is extruded.

14. Method according to any of claims 9 to 13 wherein the insert has the shape of a cylinder, an elliptical cylinder, preferably said cylinder having a diameter of less than 2 mm and a length of less than 7 mm, said elliptical cylinder having a length of less than 7 mm and the major axis of less than 2 mm.

15. Method according to any of the preceding claims 9 to 14 comprising an additional step of sterilising the insert by gamma irradiation.

## Patentansprüche

1. Bioresorbierbare ophthalmische Einlage, Folgendes umfassend:
- Hydroxypropylcellulose (HPC), wobei die HPC eine Molmasse zwischen 800.000 und 1.200.000 Da aufweist, und
- Natriumhyaluronat, wobei das Natriumhyaluronat eine Partikelgröße aufweist, die durch einen D90 von höchstens 100 µm gekennzeichnet ist.

2. Ophthalmische Einlage nach Anspruch 1, wobei das Natriumhyaluronat vernetztes Natriumhyaluronat ist.

3. Ophthalmische Einlage nach einem der vorhergehenden Ansprüche, wobei die ophthalmische Einlage das Natriumhyaluronat in einer Menge zwischen 2 und 8 Gew.-% der Einlage umfasst.

4. Ophthalmische Einlage nach einem der vorhergehenden Ansprüche, ein zweites Cellulosederivat umfassend, das sich von der ersten HPC mit einer Molmasse zwischen 800.000 und 1.200.000 Da unterscheidet, wobei das zweite Cellulosederivat aus HPC mit einer anderen Molmasse, Hydroxypropylmethylcellulose (HPMC), Carboxymethylcellulose (CMC), Methylcellulose und Mischungen daraus ausgewählt ist.

5. Ophthalmische Einlage nach einem der vorhergehenden Ansprüche, wobei die ophthalmische Einlage ein funktionales Molekül umfasst, das aus der Gruppe ausgewählt ist, die aus L-Carnitin, Betain, Trehalose, Erythritol und Mischungen daraus besteht.

6. Ophthalmische Einlage nach einem der vorhergehenden Ansprüche, wobei die ophthalmische Einlage Trehalose umfasst.

7. Ophthalmische Einlage nach einem der vorhergehenden Ansprüche, wobei die ophthalmische Einlage Natriumhyaluronat in einer Menge zwischen 2 und 8 Gew.-% der Einlage und Trehalose in einer Menge zwischen 10 und 30 Gew.-% der Einlage umfasst.

8. Ophthalmische Einlage nach einem der Ansprüche 1 bis 7 zur Verwendung bei der Prävention oder Behandlung eines Trockene-Augen-Syndroms.

9. Verfahren zur Herstellung einer ophthalmischen Einlage, wobei eine Extrusion einer Mischung, umfassend Hydroxypropylcellulose (HPC), wobei die HPC eine Molmasse zwischen 800.000 und 1.200.000 Da aufweist, und Natriumhyaluronat, wobei das Natriumhyaluronat eine Partikelgröße aufweist, die durch einen D90 von höchstens 100 µm gekennzeichnet ist, durchgeführt wird, wodurch ein Extrudat erzielt wird, und wobei das Extrudat geschnitten wird, um ophthalmische Einlagen gewünschter Abmessungen zu erhalten.

10. Verfahren nach Anspruch 9, wobei die Extrusion die folgenden Schritte umfasst:
a. Durchführen einer ersten Extrusion einer Mischung, umfassend Hydroxypropylcellulose (HPC), wobei die HPC eine Molmasse zwischen 800.000 und 1.200.000 Da aufweist, und Natriumhyaluronat, wobei das Natriumhyaluronat eine Partikelgröße aufweist, die durch einen D90 von höchstens 100 µm gekennzeichnet ist, wodurch ein Extrudat erzielt wird, und
b. Durchführen einer zweiten Extrusion des Extrudats.

11. Verfahren nach Anspruch 10, wobei die Verweildauer des Produkts während des ersten Extrusionsschritts kleiner als die Verweildauer während des zweiten Extrusionsschritts ist.

12. Verfahren nach den Ansprüchen 10 bis 11, wobei das nach dem ersten Extrusionsschritt erzielte Extrudat vor dem zweiten Extrusionsschritt pelletiert wird.

13. Verfahren nach den Ansprüchen 9 bis 11, wobei mindestens 25 Gew.-% Hydroxypropylcellulose (HPC) und zwischen 2 und 8 Gew.-% Natriumhyaluronat extrudiert werden.

14. Verfahren nach einem der Ansprüche 9 bis 13, wobei die Einlage die Form eines Zylinders, eines elliptischen Zylinders, aufweist, der Zylinder vorzugsweise einen Durchmesser von weniger als 2 mm und eine Länge von weniger als 7 mm aufweist, der elliptische Zylinder eine Länge von weniger als 7 mm und die Hauptachse von weniger als 2 mm aufweist.

15. Verfahren nach einem der vorhergehenden Ansprüche 9 bis 14, einen zusätzlichen Schritt des Sterilisierens der Einlage durch Gammabestrahlung umfassend.

## Revendications

1. Insert ophtalmique biorésorbable comprenant :
- de l'hydroxypropylcellulose (HPC), ladite HPC ayant un poids moléculaire compris entre 800 000 et 1200 000 Da, et
- du hyaluronate de sodium, dans lequel ledit hyaluronate de sodium a une taille de particule **caractérisée par** un D90 d'au plus 100 µm.

2. Insert ophtalmique selon la revendication 1, dans lequel le hyaluronate de sodium est un hyaluronate de sodium réticulé.

3. Insert ophtalmique selon l'une quelconque des revendications précédentes, dans lequel l'insert ophtalmique comprend ledit hyaluronate de sodium, en une quantité comprise entre 2 et 8 % en poids de l'insert.

4. Insert ophtalmique selon l'une quelconque des revendications précédentes, comprenant un second dérivé de cellulose, différent de la première HPC ayant un poids moléculaire compris entre 800 000 et 1200 000 Da, ledit second dérivé de cellulose étant choisi parmi HPC de poids moléculaire différent, hydroxypropylméthylcellulose (HPMC), carboxyméthylcellulose (CMC), méthylcellulose et leurs mélanges.

5. Insert ophtalmique selon l'une quelconque des revendications précédentes, dans lequel l'insert ophtalmique comprend une molécule fonctionnelle choisie dans le groupe constitué par L-carnitine, bétaïne, tréhalose, érythritol et leurs mélanges.

6. Insert ophtalmique selon l'une quelconque des revendications précédentes, dans lequel l'insert ophtalmique comprend du tréhalose.

7. Insert ophtalmique selon l'une quelconque des revendications précédentes, dans lequel l'insert ophtalmique comprend du hyaluronate de sodium, en une quantité comprise entre 2 et 8 % en poids de l'insert, et du tréhalose, en une quantité comprise entre 10 et 30 % en poids de l'insert.

8. Insert ophtalmique selon l'une quelconque des revendications 1 à 7 destiné à être utilisé dans la prévention ou le traitement du syndrome de l'œil sec.

9. Procédé de fabrication d'un insert ophtalmique, dans lequel on procède à l'extrusion d'un mélange comprenant de l'hydroxypropylcellulose (HPC), ladite HPC ayant un poids moléculaire compris entre 800 000 et 1200 000 Da, et du hyaluronate de sodium, ledit hyaluronate de sodium ayant une taille de particule **caractérisée par** un D90 d'au plus 100 µm, afin d'obtenir un extrudat ; et dans lequel ledit extrudat est coupé pour obtenir des inserts ophtalmiques aux dimensions souhaitées.

10. Procédé selon la revendication 9, dans lequel l'extrusion comprend l'étape consistant à :
a. effectuer une première extrusion d'un mélange comprenant de l'hydroxypropylcellulose (HPC), ladite HPC ayant un poids moléculaire compris entre 800 000 et 1200 000 Da, et du hyaluronate de sodium, ledit hyaluronate de sodium ayant une taille de particule **caractérisée par** un D90 d'au plus 100 µm, permettant ainsi d'obtenir un extrudat ; et
b. effectuer une seconde extrusion dudit extrudat.

11. Procédé selon la revendication 10, dans lequel le temps de séjour du produit au cours de la première étape d'extrusion est inférieur au temps de séjour au cours de la seconde étape d'extrusion.

12. Procédé selon les revendications 10 et 11, dans lequel l'extrudat obtenu après la première étape d'extrusion est mis en boulettes avant la seconde étape d'extrusion.

13. Procédé selon les revendications 9 à 11, dans lequel on extrude au moins 25 % en poids d'hydroxypropylcellulose (HPC) et entre 2 et 8 % en poids de hyaluronate de sodium.

14. Procédé selon l'une quelconque des revendications 9 à 13, dans lequel l'insert a la forme d'un cylindre, d'un cylindre elliptique, de préférence ledit cylindre ayant un diamètre inférieur à 2 mm et une longueur inférieure à 7 mm, ledit cylindre elliptique ayant une longueur inférieure à 7 mm et un grand axe inférieur à 2 mm.

15. Procédé selon l'une quelconque des revendications précédentes 9 à 14 comprenant une étape supplémentaire de stérilisation de l'insert par irradiation gamma.
